# EUROPÄISCHE PATENTSCHRIFT

(11) **EP 0 571 864 B1**
(45) Veröffentlichungstag und Bekanntmachung des Hinweises auf die Patenterteilung: **22.04.1998**
(21) Anmeldenummer: 93108034.5
(22) Anmeldetag: 17.05.1993
(51) Int. Cl.: C07C 51/235, C07C 51/25

(54) **Verfahren zur Herstellung von alpha,beta-ungesättigten Carbonsäuren**
Process for the preparation of alpha,beta unsaturated carboxylic acids
Procédé pour la préparation d'acides carboxyliques alpha,bêta-insaturés

(30) Priorität: 29.05.1992 DE 4217718
(43) Veröffentlichungstag der Anmeldung: 01.12.1993
(73) Patentinhaber: BAYER AG, 51368 Leverkusen (DE)
(72) Erfinder: Fiege, Helmut, Dr., W-5090 Leverkusen (DE); Schnatterer, Albert, Dr., W-5090 Leverkusen (DE); Littmann, Martin, Dr., W-5000 Köln 80 (DE)

(56) Entgegenhaltungen:
- DE-A- 1 443 718
- FR-A- 2 325 628
- GB-A- 782 430
- JOURNAL OF THE AMERICAN CHEMICAL SOCIETY., Bd.90, Nr.20, 25. September 1968, GASTON, PA US Seiten 5616 - 5617 E.J. COREY ET AL. 'New methods for the oxidation of aldehydes to carboxylic acids and esters'
- JOURNAL OF CHEMICAL EDUCATION, Bd.45, Nr.8, August 1968 Seiten 546 - 547 SANDRA C. THOMASON ET AL. 'Acids as derivatives of aldehydes prepared with silver oxides'
- TETRAHEDRON LETTERS, Bd.5, Februar 1967, OXFORD GB Seiten 415 - 422 J.B. LEE ET AL. 'Silver II complexes as oxidising agents'

## Beschreibung

Die vorliegende Erfindung betrifft ein Verfahren zur Oxidation von α,β-ungesättigten Aldehyden zu den entsprechenden Carbonsäuren durch katalytische Umsetzung mit Sauerstoff.

α,β-ungesättigte Carbonsäuren sind wichtige Zwischenprodukte, z.B. für Polymere, Pestizide und Pharmazeutika. Wichtige α,β-ungesättigte Carbonsäuren sind beispielsweise die Acrylsäure, die Crotonsäure und die 2-Ethylcrotonsäure, die aus den entsprechenden Aldehyden durch Oxidation mit Sauerstoff entweder in der Gasphase oder in der Flüssigphase hergestellt werden können (zur Oxidation von Acrylsäure und Crotonsäure siehe Ullmanns Encyclopädie der technischen Chemie, 4. Auflage, Band 7, S. 80 und Band 9, S. 145). Bei der Flüssigphasenoxidation von Crotonaldehyd zu Crotonsäure erreicht die Ausbeute nur 70 % der Theorie bei Umsätzen von weniger als 50 %.

Eine Variante der Flüssigphasenoxidation ist die Oxidation mit Sauerstoff in wäßrigem Medium in Gegenwart von Hydroxylionen und Silber, beschrieben beispielsweise in GB-PS 740 005, DE-OS 2 364 044 und Zhurnal Prikladnoi Khimii, Vol. 39, 2101 (1966)). Das Verfahren ist für die Herstellung eines breiten Spektrums von α,β-ungesättigten Carbonsäuren verwendbar, hat aber den Nachteil, daß viel Katalysator benötigt wird. Gemäß der GB-PS 782 430, Beispiel 11, werden für die Herstellung von 2-Ethylcrotonsäure ca. 20 Gew.-% Silber bezogen auf eingesetzten 2-Ethylcrotonaldehyd benötigt. Das Silber wird in diesem Verfahren durch Reduktion von Silberverbindungen, meist Silberoxid Ag₂O, hergestellt.

Die Reaktivierung des inaktiv gewordenen Silberkatalysators ist aufwendig. Sie geschieht normalerweise durch Lösen in Salpetersäure, Isolierung des Nitrats, Fällung als Ag₂O und anschließende Reduktion.

Es besteht deshalb das Bedürfnis nach einer Verbesserung der katalytischen Aktivität des Silbers mit dem Ziel, einer Verringerung der Katalysator-Einsatzmenge und einer Erhöhung der Standzeit, bei hoher Selektivität und hoher Reaktionsgeschwindigkeit.

Es wurde nun ein Verfahren zur Herstellung von α,β-ungesättigten Carbonsäuren der Formel 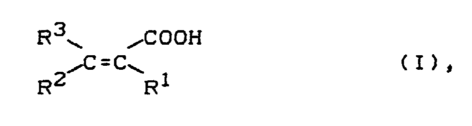 in der
- R¹und R²: unabhängig voneinander Wasserstoff, C₁-C₆-Alkyl oder C₃-C₆-Cycloalkyl und
- R³: Wasserstoff, Methyl oder Ethyl bedeuten,
durch katalytische Oxidation von α,β-ungesättigten Aldehyden der Formel (II) 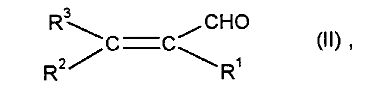 in der
- R¹, R² und R³: die bei Formel (I) angegebene Bedeutung haben,
mit Sauerstoff in Gegenwart von Wasser und Basen gefunden, das dadurch gekennzeichnet ist, daß man Silber (I,III)-oxid (AgO) zusetzt.

Überraschenderweise ergibt der Einsatz von Silber Silber(I,III)-oxid (AgO) einen außerordentlich aktiven Katalysator, mit dem Ausbeuten an α,β-ungesättigten Carbonsäuren von über 90 % bei quantitativem Umsatz möglich sind. Die Steigerung in der katalytischen Aktivität manifestiert sich auch in einer deutlich erhöhten Reaktionsgeschwindigkeit. Setzt man dagegen Silber in Form von Silbernitrat oder Silber(I)-oxid (Ag₂O) ein, so verläuft die Oxidation im Vergleich dazu langsam und unselektiv mit einer Ausbeute von ca. 40 %.

Soweit in den Formeln (I) und (II) R¹ und R² für C₁-C₆-Alkyl stehen 1, können die Alkylreste geradkettig oder verzweigt sein. Beispielsweise kommen in Frage: Methyl, Ethyl, n-Propyl, Isopropyl, n-Butyl, Isobutyl, tert.-Butyl und die verschiedenen isomeren Pentyle und Hexyle. Soweit in den Formeln (I) und (II) R¹ und R² für C₃-C₆-Cycloalkyl stehen sind Cyclopentyl und Cyclohexyl bevorzugt.

In den Formeln (I) und (II) stehen R¹ und R² vorzugsweise unabhängig voneinander für Wasserstoff oder geradkettiges oder verzweigtes C₁-C₆-Alkyl und R³ vorzugsweise für Wasserstoff.

Besonders bevorzugt stehen in den Formeln (I) und (II) R¹ und R² unabhängig voneinander jeweils für einen C₁-C₄-Alkylrest.

Ganz besonders bevorzugt wird erfindungsgemäß aus 2-Ethylcrotonaldehyd 2-Ethylcrotonsäure hergestellt.

Das erfindungsgemäß zuzusetzende Silber(I,III)-oxid (AgO) kann z.B. durch Oxidation von Silber(I)-verbindungen, wie Ag₂O oder AgNO₃, mit geeigneten Oxidationsmitteln hergestellt werden. Solche Oxidationsmittel können beispielsweise Ozon, Kaliumpermanganat, Peroxodisulfate, Hypochlorite, Anodenstrom oder Sauerstoff in Gegenwart von Sulfiten sein (siehe z.B. Gmelin, 8. Auflage, Silber Teil B1,S. 108 und Angew. Chem. 103, 1017 (1991)). Für das erfindungsgemäße Verfahren ist die Herkunft und das Herstellungsverfahren des Silber (I,III)-oxids (AgO) nicht kritisch. Ein vorteilhaftes Verfahren zur Herstellung von Silber (I,III)-oxid (AgO) ist die Oxidation von Silbernitrat mit Kaliumperoxodisulfat in wäßrig-alkalischer Lösung (siehe Gmelin, 8. Auflage, Silber Teil B1, S. 109). Dieses Verfahren ist einfach und liefert Silber (I,III)-oxid (AgO) mit hoher Ausbeute und guter Reinheit.

Das Silber (I,III)-oxid (AgO) kann in das erfindungsgemäße Verfahren trocken oder in Form einer Suspension in einer organischen oder anorganischen Flüssigkeit eingesetzt werden. Für den Fall des Einsatzes einer Suspension sind wäßrige Suspensionen bevorzugt.

Das Silber (I,III)-oxid kann man beispielsweise in Mengen von 0,1 bis 30 mol.-%, insbesondere 2 bis 12mol-%, bezogen auf den Aldehyd der Formel (II) einsetzen. Auch größere Silber (I,III)-oxid-Mengen als 30 mol-%, bezogen auf den Aldehyd der Formel (II), können ohne Nachteil für die Reaktion eingesetzt werden, sind jedoch aus wirtschaftlichen Erwägungen nicht vorteilhaft.

Das Silber (I,III)-oxid (AgO) wird unter den Bedingungen des erfindungsgemäßen Verfahrens zu elementarem Silber reduziert, der während der erfindungsgemäßen Umsetzung vorliegenden Form des Katalysators. Es ist daher sehr überraschend, daß die Reduktion von Silber (I,III)-oxid (AgO) einen wesentlich aktiveren Katalysator für die Oxidation von α,β-ungesättigten Aldehyden zu den entsprechenden Carbonsäuren ergibt als die bekannte Reduktion üblicher Silberverbindungen wie Silber(I)-oxid (Ag₂O) und Silbernitrat.

Silber (I,III)-oxid (AgO) kann in das erfindungsgemäße Verfahren auch in Kombination mit einem Trägermaterial eingesetzt werden, beispielsweise aufgebracht auf Aktivkohle, Titandioxid oder Zirkonoxid.

Als Basen für die erfindungsgemäße Umsetzung kommen z.B. Alkali- und Erdalkalihydroxide wie Lithiumhydroxid, Natriumhydroxid, Kaliumhydroxid, Magnesiumhydroxid, Calciumhydroxid, Strontiumhydroxid und Bariumhydroxid in Frage. Bevorzugt sind Natriumhydroxid und Kaliumhydroxid. Die Einsatzmenge der Base kann so bemessen werden, daß während der gesamten Oxidation ein pH-Wert von >10 aufrecht erhalten bleibt. Dazu ist eine Basenmenge von mehr als 1 Äquivalent, bezogen auf umgesetzten Aldehyd der Formel (II), notwendig. Die Base kann beispielsweise in Form einer wäßrigen Lösung einer Konzentration im Bereich von 2 bis 30 Gew.-% eingesetzt werden. Vorzugsweise beträgt die Konzentration der eingesetzten wäßrigen Base 5 bis 20 Gew.-%.

Das Verhältnis von α,β-ungesättigtem Aldehyd zu Wasser kann innerhalb eines großen Bereiches variiert werden. Wird der gesamte Aldehyd zu Beginn der Reaktion vorgelegt, ist es im allgemeinen vorteilhaft relativ viel Wasser zugegen zu haben. Wird dagegen der Aldehyd während der Reaktion erst zudosiert, so kann man mit weniger Wasser auskommen. Das Gewichtsverhältnis von Wasser zu der Summe von im Reaktionsgemisch vorliegendem α,β-ungesättigtem Aldehyd und der entsprechenden Säure kann beispielsweise im Bereich von 2 bis 25 liegen. Bevorzugt liegt es im Bereich von 3 bis 15. Es ist nicht notwendig, daß das Reaktionsgemisch eine einheitliche Phase bildet, insbesondere beim Einsatz höherer α,β-un-gesättigter Aldehyde sind auch 2-phasige Reaktionsgemische möglich.

Die Reinheit der eingesetzten Aldehyde (Verbindungen der Formel (II)) ist unkritisch. Sie können in Form der Rohprodukte eingesetzt werden, wie sie beispielsweise bei der technischen Herstellung durch Aldolkondensation oder Oxosynthese anfallen. Es können auch Gemische verschiedener Verbindungen der Formel (II) eingesetzt werden.

Bei einer besonderen Ausführungsform des erfindungsgemäßen Verfahrens wird in Gegenwart von Emulgatoren gearbeitet. Eine Übersicht über in Frage kommende Emulgatoren befindet sich in Ullmanns Encyclopedia of Industrial Chemistry, Band 8, Seite 297. Beispielhaft seien genannt; Polyglykolether und Polypropylenglykolether von Alkoholen und Phenolen, Alkylsulfonate, Alkylarylsulfonate, Alkylsulfate und quartärnere Ammoniumverbindungen. Das Arbeiten in Gegenwart eines Emulgators bewirkt häufig eine Erhöhung der Reaktionsgeschwindigkeit und der Ausbeute verglichen mit der Arbeitsweise ohne Emulgator. Der Emulgator kann z.B. in einer Menge von 0,00001 bis 0,02 Gew.-Teilen, bevorzugt 0,0001 bis 0,002 Gew.-Teilen, bezogen auf das gesamte Reaktionsgemisch, eingesetzt werden.

Da der erfindungsgemäß einzusetzende Katalysator eine hohe Reaktivität aufweist, kann die erfindungsgemäße Oxidation von Aldehyden der Formel (II) zu Carbonsäuren der Formel (I) unter thermisch schonenden Bedingungen durchgeführt werden. Bevorzugt sind Reaktionstemperaturen im Bereich von 0 bis 100°C, besonders bevorzugt solche im Bereich von 0 bis 60°C.

Als Sauerstoff für das erfindungsgemäße Verfahren kann reiner Sauerstoff (so wie er im Handel erhältlich ist) oder Sauerstoff in verdünnter Form, beispielsweise in Form von Sauerstoff enthaltenden Gasgemischen, eingesetzt werden. Die wirtschaftlich günstigste Form der Sauerstoffzugabe ist der Einsatz von atmosphärischer Luft. Der Druck des Sauerstoffs bzw. des Sauerstoff enthaltenden Gases ist nicht kritisch. Er kann z.B. zwischen 0,5 und 30 bar, vorzugsweise zwischen 0,8 und 15 bar liegen.

Bei der Verwendung von Sauerstoff enthaltenden Gasen ist deren Sauerstoffgehalt nicht kritisch. Der Gehalt und die Menge an Sauerstoff kann z.B. so eingestellt werden, daß gerade soviel Sauerstoff zugeführt wird, wie abreagiert. Es kann auch mit überschüssigem Sauerstoff gearbeitet werden. Zweckmäßigerweise achtet man darauf, daß vor, während und nach der erfindungsgemäßen Reaktion keine explosionsfähigen Gasgemische vorliegen.

Es ist von Vorteil, den Sauerstoff im Reaktionsgemisch fein zu verteilen, beispielsweise mittels Fritten. Der Sauerstoff kann auch durch kräftiges Rühren in das Reaktionsgemisch eingezogen werden.

Das erfindungsgemäße Verfahren kann kontinuierlich oder diskontinuierlich betrieben werden. Bei der diskontinuierlichen Fahrweise können die Einsatzkomponenten außer Sauerstoff vor Beginn vollständig vorgelegt werden. Es kann auch vorteilhaft sein, eine oder mehrere Komponenten, beispielsweise die Base, das Wasser oder den Aldehyd der Formel (II), vollständig vorzulegen und die noch fehlenden Komponenten während der bereits ablaufenden Reaktion zuzudosieren. Werden mehrere Komponenten bei laufender Reaktion zudosiert, so kann dies beispielsweise in Form einer Simultandosierung geschehen.

Die Aufarbeitung des Reaktionsgemisches und die Isolierung der hergestellten α,β-ungesättigten Carbonsäure der Formel (I) kann beispielsweise so erfolgen, daß man aus dem Reaktionsgemisch zunächst den Katalysator abtrennt, z.B. durch Filtration, Zentrifugieren oder Abdekantieren. Der abgetrennte Katalysator kann direkt für weitere Ansätze verwendet werden. Eine erneute Aktivierung ist nicht notwendig. Die nach der Katalysatorabtrennung verbleibende alkalische Lösung enthält dann die hergestellte α,β-ungesättigte Carbonsäure der Formel (I) in Form eines Salzes. Die freie Säure kann durch Ansäuern, beispielsweise mit Salzsäure, Schwefelsäure oder Phosphorsäure, freigesetzt und durch Phasentrennung von der wäßrigen Lösung abgetrennt werden. Man kann die Abtrennung der hergestellten α,β-ungesättigten Carbonsäure der Formel (I) gegebenenfalls auch unter Zuhilfenahme eines Extraktionsmittels durchführen.

### Beispiele

### Beispiel 1 (Katalysatorherstellung)

In 330 ml Wasser von 85°C wurden nacheinander 9,0 g Natriumhydroxid, eine Aufschlämmung von 13,5 g Kaliumperoxodisulfat in Wasser und 17,0 g Silbernitrat gelöst in möglichst wenig Wasser eingetragen. Nach 15-minütigem Rühren bei 90°C wurde der schwarze Niederschlag abgesaugt, mit Wasser gewaschen und an der Luft getrocknet. So wurden 11,4 g (= 92 % der Theorie) Silber(I,III)-oxid (AgO) in Form eines schwarzen Pulvers erhalten.

### Beispiel 2

In einem Reaktionsgefäß aus Glas wurden 2,0 g des gemäß Beispiel 1 erhaltenen Silber(I,III)-oxids (AgO), 70 ml Wasser, 0,5 g 3-Benzyl-4-hydroxybiphenyl-polyglykolether (= Emulgator) und 23,4 g 2-Ethylcrotonaldehyd (84 %ig) vorgelegt. Unter Begasung mit Sauerstoff wurde eine Lösung von 12 g Natriumhydroxid in 50 ml Wasser parallel zur Sauerstoffaufnahme so zugefügt, daß die Alkalidosierung nach Aufnahme von ca. zwei Dritteln des stöchiometrischen Sauerstoffbedarfs vollständig war. Die Reaktionstemperatur wurde bei 15°C gehalten. Nach der Aufnahme von 2,5 l Sauerstoff und einer Reaktionszeit von 5 Stunden war die Sauerstoffaufnahme beendet.

Anschließend wurde der Katalysator über eine Nutsche abfiltriert, mit Wasser und Methylenchlorid ausgewaschen und an der Luft getrocknet. Es blieben 1,78 g trockener Katalysator zurück, der für weitere Umsetzungen direkt weiterverwendet werden kann. Das Filtrat wurde nach Ansäuern bis zum Erreichen eines pH-Wertes von 1 zweimal mit Methylenchlorid extrahiert. Die Methylenchloridphase wurde über Natriumsulfat getrocknet, im Vakuum eingedampft (100 mbar, 30°C) und der ölige Rückstand gaschromatographisch analysiert.

Der eingesetzte 2-Ethylcrotonaldehyd war quantitativ umgesetzt und 2-Ethylcrotonsäure in einer Ausbeute von 92,9 % der Theorie erhalten worden.

### Beispiele 3 bis 7

Es wurde jeweils verfahren wie in Beispiel 2, jedoch wurde jeweils der aus dem vorangegangenen Beispiel zurückgewonnene Katalysator eingesetzt. Bei Beispiel 3 wurde mit den 1,78 g Katalysator aus der Aufarbeitung von Beispiel 2 begonnen. Die Ergebnisse sind in der folgenden Tabelle zusammengefaßt.

| Beispiel Nr. | Katalysatormenge (g) | Ausbeute an 2-Ethylcrotonsäure (% der Theorie) |
|---|---|---|
| 3 | 1,78 | 81,8 |
| 4 | 1,74 | 80,0 |
| 5 | 1,71 | 78,6 |
| 6 | 1,68 | 82,0 |
| 7 | 1,66 | 79,3 |

### Beispiel 8 (Vergleichsbeispiel)

Es wurde verfahren wie in Beispiel 2, jedoch wurden 2,0 g Silber(I)-oxid (Ag₂O) eingesetzt, das durch Versetzen einer wäßrigen Silbernitratlösung mit wäßriger Natronlauge erhalten worden war. Nach 8 Stunden Reaktionszeit waren 3,1 1 Sauerstoff aufgenommen worden. Die gaschromatographische Analyse des bei der Aufarbeitung angefallenen öligen Rückstandes ergab, daß sich 2-Ethylcrotonsäure in einer Ausbeute von 38,5 % der Theorie gebildet hatte.

## Patentansprüche

1. Verfahren zur Herstellung von α,β-ungesättigten Carbonsäuren der Formel (I) 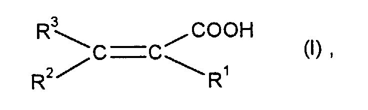 in der
R¹ und R² unabhängig voneinander Wasserstoff, C₁-C₆-Alkyl oder C₃-C₆-Cycloalkyl und
R³ Wasserstoff, Methyl oder Ethyl bedeutet,
durch katalytische Oxidation von α,β-ungesättigten Aldehyden der Formel (II) 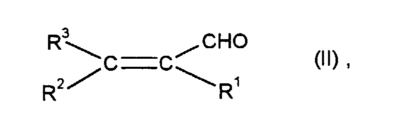 in der
R¹, R² und R³ die bei Formel (I) angegebene Bedeutung haben,
mit Sauerstoff in Gegenwart von Wasser und Basen, dadurch gekennzeichnet, daß man Silber Silber(I,III)-oxid (AgO) zusetzt.

2. Verfahren nach Anspruch 1, dadurch gekennzeichnet, daß in den Formeln (I) und (II) R¹ und R² unabhängig voneinander für Wasserstoff oder geradkettiges oder verzweigtes C₁-C₆-Alkyl und R³ für Wasserstoff stehen.

3. Verfahren nach Ansprüchen 1 und 2, dadurch gekennzeichnet, daß man das Silber(I,III)-oxid durch Oxidation von Silber(I)-Verbindungen mit Ozon, Kaliumpermanganat, Peroxodisulfaten, Hypochloriten, Anodenstrom oder Sauerstoff in Gegenwart von Sulfiten hergestellt hat.

4. Verfahren nach Ansprüchen 1 bis 3, dadurch gekennzeichnet, daß man 0,1 bis 30 Mol-% Silber(I,III)oxid (AgO) bezogen auf den Aldehyd der Formel (II) einsetzt.

5. Verfahren nach Ansprüchen 1 bis 4, dadurch gekennzeichnet, daß man es bei Temperaturen im Bereich 0 bis 100°C durchführt.

6. Verfahren nach Ansprüchen 1 bis 5, dadurch gekennzeichnet, daß man Sauerstoff in Form von atmosphärischer Luft zuführt.

7. Verfahren nach Ansprüchen 1 bis 6, dadurch gekennzeichnet, daß man als Basen Alkali- und/oder Erdalkalihydroxide in einer solchen Menge zufügt, daß während der gesamten Oxidation ein pH-Wert von größer als 10 aufrecht erhalten bleibt.

8. Verfahren nach Ansprüchen 1 bis 7, dadurch gekennzeichnet, daß das Gewichtsverhältnis von Wasser zu der Summe aus α,β-ungesättigtem Aldehyd und der entsprechenden Säure im Bereich von 2 bis 25 liegt.

9. Verfahren nach Ansprüchen 1 bis 8, dadurch gekennzeichnet, daß man in Gegenwart eines Emulgators arbeitet.

10. Verfahren nach Ansprüchen 1 bis 9, dadurch gekennzeichnet, daß man nach Beendigung der Oxidation den Katalysator abtrennt und für weitere Ansätze wiederverwendet.

## Claims

1. Process for the preparation of α,β-unsaturated carboxylic acids of the formula (I) 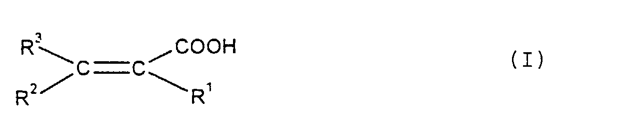 in which
R¹ and R² independently of one another denote hydrogen, C₁-C₆-alkyl or C₃-C₆-cycloalkyl and
R³ denotes hydrogen, methyl or ethyl,
by catalytic oxidation of α,β-unsaturated aldehydes of the formula (II) 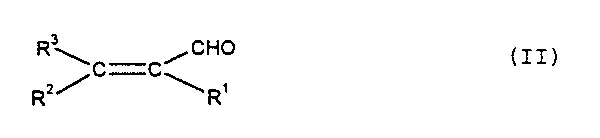 in which
R¹, R² and R³ have the meaning given in the case of formula (I),
with oxygen in the presence of water and bases, characterised in that silver(I,III) oxide (Ago) is added.

2. Process according to Claim 1, characterised in that, in the formulae (I) and (II), R¹ and R² independently of one another represent hydrogen or straight-chain or branched C₁-C₆-alkyl and R³ represents hydrogen.

3. Process according to Claims 1 and 2, characterised in that the silver(I,III) oxide has been prepared by oxidation of silver(I) compounds with ozone, potassium permanganate, peroxodisulphates, hypochlorites, an anodic current or oxygen in the presence of sulphites.

4. Process according to Claims 1 to 3, characterised in that 0.1 to 30 mol % of silver(I,III) oxide (AgO), based on the aldehyde of the formula (II), is employed.

5. Process according to Claims 1 to 4, characterised in that it is carried out at temperatures in the range from 0 to 100°C.

6. Process according to Claims 1 to 5, characterised in that oxygen is added in the form of atmospheric air.

7. Process according to Claims 1 to 6, characterised in that alkali metal hydroxides and/or alkaline earth metal hydroxides in an amount such that a pH of greater than 10 is maintained throughout the entire oxidation are added as the bases.

8. Process according to Claims 1 to 7, characterised in that the weight ratio of water to the total of α,β-unsaturated aldehyde and the corresponding acid is in the range from 2 to 25.

9. Process according to Claims 1 to 8, characterised in that it is carried out in the presence of an emulsifier.

10. Process according to Claims 1 to 9, characterised in that, when the oxidation has ended, the catalyst is separated off and is reused for further batches.

## Revendications

1. Procédé de préparation d'acides carboxyliques α,β-insaturés de formule 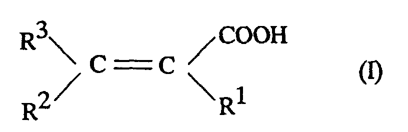 où
R¹ et R² représentent indépendamment l'un de l'autre l'hydrogène, un alkyle en C₁-C₆ ou un cycloalkyle en C₃-C₆ et
R³ représente l'hydrogène, un méthyle ou un éthyle,
par oxydation catalytique d'aldéhydes α,β-insaturés de formule (II) 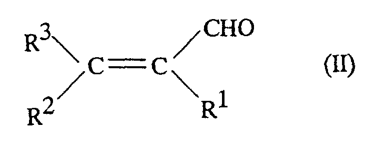 où
R¹, R² et R³ ont la signification indiquée pour la formule (I),
avec de l'oxygène en présence d'eau et de bases, caractérisé en ce que l'on ajoute de l'oxyde d'argent (I,III) (AgO).

2. Procédé selon la revendication 1, caractérisé en ce que, dans les formules (I) et (II), R¹ et R² représentent indépendamment l'un de l'autre l'hydrogène ou un alkyle en C₁-C₆ linéaire ou ramifié et R³ représente l'hydrogène.

3. Procédé selon les revendications 1 et 2, caractérisé en ce que l'on a préparé l'oxyde d'argent (I), (III) par oxydation de composés de l'argent (I) avec l'ozone, le permanganate de potassium, des peroxodisulfates, des hypochlorites, un courant anodique ou l'oxygène en présence de sulfites.

4. Procédé selon les revendications 1 à 3, caractérisé en ce que l'on utilise 0,1 à 30 mol % d'oxyde d'argent (I,III) (AgO) par rapport à l'aldéhyde de formule (II).

5. Procédé selon les revendications 1 à 4, caractérisé en ce que l'on opère à des températures dans le domaine de 0 à 100°C.

6. Procédé selon les revendications 1 à 5, caractérisé en ce que l'on introduit l'oxygène sous forme d'air atmosphérique.

7. Procédé selon les revendications 1 à 6, caractérisé en ce que l'on ajoute comme bases des hydroxydes alcalins et/ou alcalino-terreux en une quantité telle qu'un pH supérieur à 10 soit maintenu pendant toute l'oxydation.

8. Procédé selon les revendications 1 à 7, caractérisé en ce que le rapport massique de l'eau à la somme de l'aldéhyde α,β-insaturé et de l'acide correspondant est situé dans le domaine de 2 à 25.

9. Procédé selon les revendications 1 à 8, caractérisé en ce que l'on opère en présence d'un émulsifiant.

10. Procédé selon les revendications 1 à 9, caractérisé en ce que, après la fin de l'oxydation, on sépare le catalyseur et on le réutilise pour d'autres opérations.
